# EUROPEAN PATENT SPECIFICATION

(11) **EP 3 280 385 B1**
(45) Date of publication and mention of the grant of the patent: **29.09.2021**
(21) Application number: 16728185.6
(22) Date of filing: 25.05.2016
(51) Int. Cl.: A61K 8/44, A61K 8/46, A61Q 11/00, A61K 8/19, A61K 8/24

(54) **FOAMING DENTIFRICE WITH DESENSITIZING AGENTS**
SCHÄUMENDE ZAHNPASTE MIT DESENSIBILISIERUNGS-AGENS
DENTIFRICE MOUSSANTE AVEC AGENT POUR LA DESENSIBILISATION

(30) Priority: 29.05.2015 US 201562168048 P
(43) Date of publication of application: 14.02.2018
(73) Proprietor: Colgate-Palmolive Company, New York, NY 10022 (US)
(72) Inventor: POTH, Tilo, 69469 Weinheim (DE); WOLF, Hanspeter, 5076 Bozen (CH); SCHMID, Joshua, 4242 Laufen (CH); D'AMBROGIO, Robert, Princeton, New Jersey 08540 (US)
(74) Representative: Wibbelmann, Jobst
(86) International application number: PCT/US2016/034142
(87) International publication number: WO 2016/196131

(56) References cited:
- EP-A1- 1 660 028
- EP-A1- 2 281 543
- WO-A1-2010/114549
- WO-A1-2013/094312
- WO-A2-98/23249
- JP-A- 2006 104 101
- JP-A- 2011 256 125
- Anonymous: "GNPD - Anti-Sensitivity Toothpaste", , 1 August 2011 (2011-08-01), XP055287816, Retrieved from the Internet: URL:http://www.gnpd.com/sinatra/recordpage /1607172/from_search/IaEOjVCwgE/?page=1 [retrieved on 2016-07-12]

## Description

### BACKGROUND

Dentinal hypersensitivity (i.e. sensitivity) is a painful condition resulting from the movement of liquid in exposed dentin tubules from external stimuli such as pressure and temperature.

"GNPD - Anti-Sensitivity Toothpaste" (GNPD No. 1607172), JP2011256125A, JP2006104101A,

EP1660028A1, WO2013/094312A1, WO2010/114549A1, EP2281543A1 and WO98/23249A2 disclose dental hygiene products. However, these documents are silent to the combinations of a specific amount of sodium lauryl sulfate (SLS), a specific amount of cocamidopropyl betaine (CAPB) and a potassium salt, but no non-ionic surfactant.

Toothpastes fighting sensitive teeth often contain the salt potassium nitrate. However, this ingredient is well known to have foam breaking properties making it difficult to obtain the sensory foam profile which delivers improved consumer acceptance. In many regions of the world consumers prefer high foaming toothpastes over products that have a poor foaming ability. Conventional levels of surfactants usually yield acceptable foam volumes when tested in pure water. However, when the testing is performed in solutions mimicking the composition of human saliva it is hard to achieve foam levels that exceed a certain volume as also human saliva has foam breaking properties.

Thus, there is a need for an oral care composition formulation which retains excellent foaming properties in human saliva when formulated with salts such as potassium nitrate, zinc salts or phosphate salts.

### BRIEF SUMMARY

The present invention relates to an oral care composition that achieves high foam levels in the presence of high salt levels (e.g., potassium nitrate, phosphates and zinc salts) by simultaneously reducing and compensating for the foam-breaking properties of saliva.

Surprisingly, the present inventors discovered that a dual surfactant system comprising an anionic surfactant, wherein the anionic surfactant is sodium lauryl sulfate (SLS) and a cationic or amphoteric surfactant, wherein the cationic or amphoteric surfactant is cocamidopropyl betaine (CAPB) that does not comprise a non-ionic surfactant, delivers improved foaming properties in artificial saliva and also compensates for the foam breaking properties of saliva. This is particularly unexpected as the dual-surfactant system produces better foaming than a triple-surfactant system having a higher total surfactant content. The system also delivers high volumes of very smooth and stable foam even in the presence of high levels of salts that usually act as foam-breakers. The invention therefore delivers pleasant foam levels independent even from combinations of foam challenging ingredients. In particular, the invention allows the formulation of oral care products with desensitizing ingredients such as potassium nitrate, with anti-tartar systems such as poly-phosphate salts and/or with antibacterial agents such as zinc compounds without loss of foaming. In addition, the foam obtained from a surfactant system according to the invention surprisingly delivers the desired foam volume much faster than conventional surfactant systems under the same conditions. The consumer therefore experiences the improved foaming properties within the timeframe that is most relevant for the application of the product. The surfactant system according to the invention is also cost efficient and shows improved micro robustness.

The present invention also relates to an oral care composition, for example a dentifrice composition, for treating or preventing hypersensitivity of the teeth and to a method of treating or preventing hypersensitivity of the teeth.

The non-ionic surfactant that is excluded from the oral care composition may be a polyoxyethylene surfactant, such as Polyoxamer 407, Steareth 30, Polysorbate 20, or castor oil.

In one embodiment, the oral care composition comprises polymers selected from cellulose derived thickening and film forming polymers (such as carboxymethyl cellulose (CMC)), polysaccharide based rheology modifiers (such as Xanthan Gum), and methyl vinyl ether/maleic acid copolymers. In addition to their viscosity and rheology building properties, the polymers also surprisingly contribute to the foam consistency and the mouth feel of the oral care composition.

### DETAILED DESCRIPTION

The present invention refers to an oral care composition comprising: an anionic surfactant, wherein the anionic surfactant is sodium lauryl sulfate (SLS); a cationic or amphoteric surfactant, wherein the cationic or amphoteric surfactant is cocamidopropyl betaine (CAPB); and a potassium salt; wherein the oral care composition does not comprise a non-ionic surfactant, wherein sodium lauryl sulfate is present in an amount of from 1.0 weight % to 3.0 weight %, and wherein cocamidopropyl betaine is present in an amount of from 0.30 weight% to 0.60 weight % of the composition. The oral care compositions of the present invention can be in the form of a dentifrice (including toothpastes, toothpowders, and prophylaxis pastes) or confectionaries (including gums, beads and chews).

Examples of further surfactants include water-soluble salts of higher fatty acid monoglyceride monosulfates, such as the sodium salt of monosulfated monoglyceride of hydrogenated coconut oil fatty acids; higher alkyl sulfates; alkyl aryl sulfonates such as sodium dodecyl benzene sulfonate; higher alkyl sulfoacetates, such as sodium lauryl sulfoacetate; higher fatty acid esters of 1,2-dihydroxypropane sulfonate; and the substantially saturated higher aliphatic acyl amides of lower aliphatic amino carboxylic compounds, such as those having 12-16 carbons in the fatty acid, alkyl or acyl radicals; and the like. Examples of the last mentioned amides include N-lauryl sarcosine, and the sodium, potassium and ethanolamine salts of N-lauryl, N-myristoyl, or N-palmitoyl sarcosine. Others include, amphoteric surfactants, such as cocamidopropyl betaine lauryl glucoside; condensation products of ethylene oxide with various hydrogen containing compounds that are reactive therewith and have long hydrocarbon chains (e.g., aliphatic chains of from 12 to 20 carbon atoms), which condensation products (ethoxamers) contain hydrophilic polyoxyethylene moieties, such as condensation products of poly (ethylene oxide) with fatty acids, fatty, alcohols, fatty amides and other fatty moieties, and with propylene oxide and polypropylene oxides.

The oral care composition comprises a dual surfactant system as defined in the claims. In one embodiment, the oral composition includes a dual surfactant system that consists of sodium lauryl sulfate (SLS) and cocamidopropyl betaine. The sodium lauryl sulfate is contained in an amount of from 1.0 to 3.0 weight %, and the cocamidopropyl betaine is contained in an amount of from 0.30 weight% to 0.60 weight % (e.g., 1.0 to 2.0 weight % of a 30 % aqueous cocamidopropyl betaine solution), based on the total weight of the oral care composition. In some embodiments, the composition comprises a single anionic surfactant and a single cationic/amphoteric surfactant, that is, the composition comprises sodium lauryl sulfate and cocamidopropyl betaine as the only surfactants. In some embodiments, the ratio of sodium lauryl sulfate to cocamidopropyl betaine is 10:1 to 5:1, or 10:1 or 6:1 or 7.5:1.

The oral care composition does not comprise a nonionic surfactant. Non-ionic surfactants that are excluded from the oral care composition may be a polyoxyethylene surfactants, such as Poloxamer 407, Steareth 30, Polysorbate 20, or castor oil. The generic term "poloxamer" refers to nonionic surfactants of the polyethylene oxide-polypropylene oxide block copolymer class. Thus, the composition is free of polyethylene oxide/polypropylene oxide block copolymers.

The oral care compositions of the invention may include one or more agents to increase the amount of foam that is produced when the oral cavity is brushed. Such foaming agents are known to those of skill in the art. Illustrative examples of agents that increase the amount of foam include, but are not limited to polyoxyethylene foaming agents and certain polymers including, but not limited to, alginate polymers. The polyoxyethylene foaming agent may increase the amount of foam and the thickness of the foam generated by the oral care carrier component of the present invention. Polyoxyethylene is also commonly known as polyethylene glycol ("PEG") or polyethylene oxide. As used herein, "polyethylene glycol" refers to a polyoxyethylene foaming agent. The amount of foaming agent in the oral care composition may be from 1 to 5 weight %; preferably from 2 to 4 weight %; preferably 3 weight %, and all ranges and sub ranges there between, based on the total weight of the oral care composition.

In one embodiment, the oral care composition comprises silica. In one embodiment the silica is an amorphous silica such as Zeodent 105, Zeodent 114 and Zeodent 165 marketed by J.M. Huber Chemicals Division, Havre de Grace, Md. 21078); Sylodent 783 and Sylodent VP5 (marketed by Davison Chemical Division of W.R. Grace & Company); or Sorbosil AC 43 (from PQ Corporation). In some embodiments, the oral care composition comprises a mixture of silicas.

In one embodiment, the silica is a precipitated silica. In one embodiment the precipitated silica has a pellicle cleaning ratio (PCR) of greater than 85 when tested at 20% loading. In one embodiment this precipitated silica also has a mean particle size d₅₀ of from 5 to 15 µm and an oil absorption of from 40 to 120 cm³/100g silica.

The cleaning efficacy of the precipitated silica is expressed using the pellicle cleaning ratio (PCR). This is typically measured at 20 % silica loading. This high cleaning silica preferably has a PCR value of greater than 85.

In one embodiment the oral care composition may comprise from 7-30 weight % total silica, preferably from 10 to 25 weight % silica, preferably from 12 to 22 weight % silica, preferably from 15 to 21 weight % silica.

The oral compositions may comprise significant levels of water. Water employed in the preparation of commercial oral compositions should be deionized and free of organic impurities. The amount of water in the compositions includes the free water which is added plus that amount which is introduced with other materials. The oral care compositions of the present invention may comprise from 5 to 50 weight % water, based on the composition, preferably from 15 to 40 weight % water, preferably from 20 to 35 weight % water, and all ranges and sub ranges there between.

Compositions of the present invention may also comprise a humectant, e.g., to prevent the composition from hardening upon exposure to air. Certain humectants can also impart desirable sweetness or flavor to dentifrice compositions. The humectant, on a pure humectant basis, generally includes 15% to 70 weight %, preferably 25 % to 50 weight %, preferably 30 to 45 weight %, and all ranges and sub ranges there between, based on the total weight of the oral care composition. Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In one embodiment, the oral composition further comprises an antibacterial agent selected from a halogenated diphenyl ether (e.g. triclosan), a magnolol derivative, an herbal extract or essential oil (e.g., rosemary extract, tea extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), a bisguanide antiseptic (e.g., chlorhexidine, alexidine or octenidine), a quaternary ammonium compound (e.g., cetylpyridinium chloride (CPC), benzalkonium chloride, tetradecylpyridinium chloride (TPC), N-tetradecyl-4-ethylpyridinium chloride (TDEPC)), a phenolic antiseptic, hexetidine, octenidine, sanguinarine, povidone iodine, delmopinol, salifluor, metal ions (e.g., zinc salts, for example, zinc citrate, a stannous salt, a copper salt, an iron salt), sanguinarine, propolis and an oxygenating agent (e.g., hydrogen peroxide, buffered sodium peroxyborate or peroxycarbonate), phthalic acid or a salt thereof, monoperthalic acid or a salt or ester thereof, ascorbyl stearate, oleoyl sarcosine, alkyl sulfate, dioctyl sulfosuccinate, salicylanilide, domiphen bromide, delmopinol, octapinol or another piperidino derivative, a nicin preparation, a chlorite salt; and a combination of two or more thereof.

In one embodiment, the oral composition comprises an antibacterial agent which is a zinc ion source. Optionally, the zinc ion source is at least one selected from zinc citrate, zinc oxide, zinc acetate, zinc gluconate, zinc glycinate, zinc sulfate, zinc phosphate and sodium zinc citrate. Further optionally, the composition comprises zinc oxide and zinc citrate.

In some embodiments, the antibacterial agent is present in an amount of 0.01% to 5 weight %; preferable 1 to 3 weight % of the total composition weight

In one embodiment, the oral care composition may optionally comprise a tartar control agent. In one embodiment the tartar control agent may be chosen from phosphates and polyphosphates, polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids, and salts of any of these agents (for example, their alkali metal and ammonium salts). In some embodiments, the tartar control agent is a monobasic, dibasic or tribasic sodium phosphate; a mono-, di- or trisodium pyrophosphate; a mono-, di- or tripotassium pyrophosphate; tetrasodium pyrophosphate (TSPP); tetrapotassium pyrophosphate (TKPP); sodium or potassium tripolyphosphate; sodium or potassium tetrapolyphosphate; sodium or potassium trimetaphosphate; sodium or potassium hexametaphosphate; or a combination of any two or more thereof. In such tartar control agents, sodium or potassium can optionally be replaced by ammonium. In some embodiments, the tartar control agent is tetrasodium pyrophosphate, tetrapotassium pyrophosphate, or a combination thereof.

In addition to controlling tartar, certain tartar controlling agents (such as, for example, pyrophosphates) can also act to stabilize any fluoride present in the compositions, and to mask the taste of zinc.

In some embodiments, the total concentration of the tartar control agent in the oral care composition is from 0.2 to 5 weight %; from 0.25 to 4 weight %; from 0.3 to 3 weight %; or from 0.4 to 2.5 weight % based on the total weight of the oral care composition. In one embodiment, the tartar control agent is tetrapotassium pyrophosphate and the total concentration of the tartar control agent is from 1 weight % to 4 weight %; from 2 weight % to 3 weight %; from 2.4 weight % to 2.6 weight %; 2.4 weight % or 2.5 weight %. In one embodiment the tartar control agent is tetrasodium pyrophosphate and the total concentration of the tartar control agent is from 0.2 to 0.8 weight %; from 0.3 to 0.7 weight %; from 0.4 to 0.6 weight %; or 0.5 weight %.

The oral care composition comprises an orally acceptable potassium salt, e.g., potassium nitrate or potassium chloride, in an amount effective to reduce dentinal sensitivity. Some embodiments comprise from 0.1% to 7.5%; preferably from 2 to 7 weight %; preferably from 4 to 6 weight %; preferably 5 weight %; of an orally acceptable potassium salt, e.g., potassium nitrate and/or potassium chloride, based on the total weight of the oral care composition.

The oral care compositions of the invention may also include additional polymers to adjust the viscosity of the formulation or enhance the solubility of other ingredients. These additional polymers may add additional body and stability to the generated foam. Such additional polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. In one embodiment these additional polymers are contained in an amount of from 0.1 to 10 weight %, based on the total weight of the composition. In one embodiment the oral care composition comprises xanthan gum in an amount from 0.1 to 1.8 weight %; preferably from 0.2 to 1.00 weight %; most preferably from 0.4 to 0.65 weight %, and all ranges and sub ranges there between, based on the total weight of the oral care composition.

Silica thickeners, which form polymeric structures or gels in aqueous media, may be present. Note that these silica thickeners are physically and functionally distinct from the particulate silica abrasives also present in the compositions. Other thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of 0.5% to 5.0% by weight of the total composition are used.

The compositions of the invention may include an anionic polymer, for example in an amount of from 0.05 to 10%, or 0.05 to 5%. Such agents include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of 30,000 to 1,000,000, most preferably 300,000 to 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The polymer may also be present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of 1,000 to 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 Sikes et al.

In any embodiments of each of the above aspects, the oral care compositions may further comprise additional ingredients. These additional ingredients may include, but are not limited to, diluents (e.g. water), bicarbonate salts, surfactants, foam modulators, sweeteners, flavorants, pigments, antibacterial agents, anticaries agents, humectants (such as sorbitol or glycerin), preservatives, thickeners (such as thickening silicas), gums (such as xanthan gum or carboxymethylcellulose (CMC)) and mixtures thereof. In one embodiment the oral care composition contains CMC from 0.3 to 1.5 %, preferably from 0.5 to 1.3 %, or 0.5% or 1.0%. In some embodiments, the oral care composition contains xanthan gum in an amount of 0.1 to 1.5%, for example, 0.5 to 1% or 0.65% by weight of the composition. In some embodiments, the composition contains both CMC and xanthan gum.

In some embodiments, the oral care composition of the invention may also contain a source of fluoride ions or a fluorine-providing ingredient in amounts sufficient to supply 25 ppm to 5,000 ppm of fluoride ions, generally at least 500 ppm, e.g., 500 to 2000 ppm, e.g., 1000 - 1600 ppm, e.g., 1000ppm, also e.g., 1450 ppm. Fluoride ion sources may be added to the compositions of the invention at a level of 0.01 weight % to 10 weight %, 0.03 weight % to 5 weight %, 0.1 weight % to 1 weight %, 0.2 to 0.8 weight%; or 0.2 to 0.4 weight % of the composition. However, it is to be understood that the weights of fluoride salts to provide the appropriate level of fluoride ion will obviously vary based on the weight of the counter ion in the salt, and one of skill in the art may readily determine such amounts. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source preferably includes stannous fluoride.

The oral care compositions of the present invention may comprise at least one sweetener (such as, for example, sodium saccharin), useful for example to enhance taste of the composition. One or more sweeteners are optionally present in a total amount depending strongly on the particular sweetener(s) selected, but typically 0.005 wt.% to 5 wt.%, by total weight of the composition, optionally 0.01 wt.% to 1 wt.%, further optionally 0.1 wt.% to 0.5 wt.% by total weight of the oral care composition.

The oral care compositions of the present invention may also comprise at least one flavorant, useful for example to enhance taste of the composition. One or more flavorants are optionally present in a total amount of from 0.01 wt. % to 5 wt. %, for example, from 0.03 wt. % to 2.5 wt.%, optionally 0.05 wt.% to 1.5 wt.%, further optionally 0.1 wt.% to 0.3 wt.% by total weight of the oral care composition

The oral care compositions of the invention may comprise at least one colorant. Colorants herein include pigments, dyes, lakes and agents imparting a particular luster or reflectivity such as pearling agents. Any orally acceptable colorant can be used. Colorants among those useful herein include FD&C Red No. 3 (sodium salt of tetraiodofluorescein), Food Red 17, disodium salt of 6-hydroxy-5- {(2-methoxy-5-methyl-4-sulphophenyl)azo} -2-naphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-methylene}-[1-(N-ethyl-N-p-sulfobenzyl)-Δ-3,5-cyclohexadienimine], FD&C Blue No. 1 (disodium salt of dibenzyldiethyl-diaminotriphenylcarbinol trisulfonic acid anhydrite), FD&C Blue No. 2(sodium salt of disulfonic acid of indigotin), and mixtures thereof in various proportions. In one embodiment, the colorant comprises a water insoluble inorganic pigment, such as titanium dioxide, chromium oxide green, phthalocyanine green, ultramarine blue, ferric oxide, or a water insoluble dye lake. In some embodiments, dye lakes include calcium or aluminum salts of an FD&C dye such as FD&C Green #1 lake, FD&C Blue #2 lake, D&C Red #30 lake or FD&C # Yellow 15 lake. In certain embodiments, a water soluble dye, such as, for example, FD&C Blue #1, is contained within a water-insoluble polymer such as, for example polyethylene such as that found in polyethylene beads (e.g., Microblue Spectrabeads, sold by Micropowders, Inc.). In certain embodiments, the oral care composition comprises a dye such as D&C Red #30. In certain embodiments, a white colorant is used, for example titanium dioxide (TiO2), titanium dioxide coated mica (e.g., Timiron), a mineral, or a clay.

One or more colorants are optionally present in a total amount of from 0.0001 wt.% to 5 wt.%, for example, from 0.0001 wt.% to 1 wt. %, or from 0.0005 wt % to 0.1 wt.%, by total weight of the oral care composition. In one embodiment the oral care composition comprises a mixture of colorants.

Optionally, the composition is a dentifrice, a toothpaste, a gel, a tooth powder, a mouthwash, a mouthrinse, a lozenge, a tablet, a spray, a gum, or a film.

The oral care compositions of the invention are useful for treating or preventing hypersensitivity of the teeth, comprising the step of contacting the oral cavity (e.g. the teeth) with said oral care composition.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

### EXAMPLES

Three examples were prepared. Foam measurements were carried out in artificial saliva using the SITA foam tester.

### Example 1: Toothpaste A

Example 1 exemplifies an oral composition with medium abrasivity containing a foam system A having 2.18 weight % SLS, 1.25 weight % of a 30 % aqueous CAPB solution (0.38 wt % CAPB) and 0.4 weight % xanthan gum, based on the total weight of the oral care composition.

**Table 1 Compositions of Toothpaste A**

| | **Toothpaste A** |
|---|---|
| | |
| GLYCERIN | 22.23 |
| WATER, FLAVOR, SWEETENER, COLORANTS | 13.49 |
| SORBITOL 70% SOLN. | 29.30 |
| POLYETHYLENE GLYCOL 600 | 3.00 |
| POTASSIUM NITRATE | 5.00 |
| SODIUM FLUORIDE | 0.32 |
| SODIUM CMC | 1.00 |
| TETRASODIUM PYROPHOSPHATE | 0.50 |
| SILICA-ABRASIVE | 8.00 |
| SILICA-THICKENER | 8.00 |
| COCAMIDOPROPYL BETAINE, 30% in water | 1.25 |
| Water /SLS slurry 29% | 7.52 |
| XANTHAN GUM | 0.40 |
| **Total Components** | **100.00000** |

The below chart illustrates the significant difference in foam volume during the first minute of the application to artificial saliva. This table compares Toothpaste A with the foam system A against a comparative composition A which has the same components as Toothpaste A, with the distinction that this comparative composition A does not comprise the foam system A (SLS + CAPB) and instead comprises a foam system consisting of 3.90 weight % of the water/SLS slurry 29% (1.13 wt% SLS; balance of difference is additional water).

**Table 2 Foam Volume Data for Toothpaste A in Artificial Saliva with and without Foam System A**

| | **Toothpaste A w/ described foam system A** | **Toothpaste A w/o described foam system A** |
|---|---|---|
| Time (sec.) | Foam volume /ml | Foam volume /ml |
| 0 | 0 | 0 |
| 10 | 192 | 158 |
| 20 | 262 | 218 |
| 30 | 345 | 266 |
| 40 | 404 | 300 |
| 50 | 412 | 332 |
| 60 | 415 | 368 |
| 70 | 419 | 381 |
| 80 | 421 | 398 |
| 90 | 422 | 407 |
| 100 | 423 | 407 |

### Example 2: Toothpaste B

Example 2 exemplifies an oral composition with greater abrasivity containing a foam system B having 2.18 weight % SLS, 1.25 weight % of a 30 % aqueous CAPB solution (0.38 wt % CAPB), and 0.65 weight % xanthan gum, based on the total weight of the oral care composition.

**Table 3 Compositions of Toothpaste B**

| | **Toothpaste B** |
|---|---|
| | |
| GLYCERIN | 26.00 |
| WATER, FLAVOR, SWEETENER, COLORANTS | 17.20 |
| SORBITOL 70% SOLN. | 17.26 |
| POLYETHYLENE GLYCOL 600 | 3.00 |
| POTASSIUM NITRATE | 5.00 |
| SODIUM FLUORIDE | 0.32 |
| SODIUM CMC | 0.50 |
| SILICA-ABRASIVE | 8.00 |
| SILICA-THICKENER | 3.00 |
| HIGH CLEANING SILICA | 10.00 |
| COCAMIDOPROPYL BETAINE, 30% in water | 1.25 |
| Water /SLS slurry 29% | 7.52 |
| XANTHAN GUM | 0.65 |
| 50% KOH | 0.30 |
| **Total Components** | **100.000000** |

The below chart illustrates the significant higher foam obtained with the foam system B according to the invention particularly during the consumer relevant first 60 seconds of the application to artificial saliva. This table compares Toothpaste B with the foam system B against a comparative composition B which has the same components as Toothpaste B, with the distinction that this comparative composition B does not comprise the foam system B (SLS/CAPB/xanthan gum) and instead comprises a foam system consisting of 3.90 weight % of the water/SLS slurry 29% (1.13 wt% SLS; balance of difference is additional water).

**Table 4 Foam Volume Data for Toothpaste B in Artificial Saliva with and without Foam System B**

| | **Toothpaste B w/ new foam system B** | **Toothpaste B w/o new foam system B** |
|---|---|---|
| Time (sec.) | Foam volume /ml | Foam volume /ml |
| 0 | 0 | 0 |
| 10 | 170 | 149 |
| 20 | 251 | 192 |
| 30 | 326 | 220 |
| 40 | 371 | 246 |
| 50 | 390 | 276 |
| 60 | 399 | 295 |
| 70 | 400 | 310 |
| 80 | 401 | 325 |
| 90 | 402 | 356 |
| 100 | 402 | 374 |

It can be seen in Figure 4 the foam generating system according to the invention results in significantly higher foaming.

### Example 3: Toothpaste C

Example 3 exemplifies an oral composition with medium abrasivity containing a foam system C having 2.18 weight % SLS, 1.25 weight % of a 30 % aqueous CAPB solution (0.38 wt % CAPB), 0.65 weight % xanthan gum, 2.44 % tetrasodium pyrophosphate and 2.00 % zinc citrate, based on the total weight of the oral care composition.

**Table 5 Compositions of Toothpaste C**

| | **Toothpaste C** |
|---|---|
| | |
| GLYCERIN | 17.00 |
| WATER, FLAVOR, SWEETENER, COLORANTS | 18.01 |
| SORBITOL - 70% SOLN. | 17.00 |
| POLYETHYLENE GLYCOL 600 | 3.00 |
| POTASSIUM NITRATE | 5.00 |
| SODIUM FLUORIDE | 0.32 |
| ZINC CITRATE TRIHYDRATE | 2.00 |
| SODIUM CMC | 0.40 |
| GANTREZ | 9.09 |
| TETRAPOTASSIUM PYROPHOSPHATE | 2.44 |
| SILICA-ABRASIVE | 8.00 |
| SILICA-THICKENER | 7.00 |
| COCAMIDOPROPYL BETAINE, 30% in water | 1.25 |
| Water /SLS slurry 29% | 7.51 |
| XANTHAN GUM | 0.65 |
| 50% KOH | 1.32 |
| **Total Components** | **100.00000** |

The below chart illustrates the significant higher foam obtained with the foam system C according to the invention particularly during the consumer relevant first 60 seconds of the application to artificial saliva. This table compares Toothpaste C with the foam system C against a comparative composition C which has the same components as Toothpaste C, with the distinction that this comparative composition C does not comprise the foam system C and instead comprises a foam system consisting of 4.91 weight % of the water/SLS slurry 29% (1.42 wt% SLS; balance of difference is additional water).

**Table 6 Foam Volume Data for Toothpaste C in Artificial Saliva with and without Foam System C**

| | **Toothpaste C w/ described foam system C** | **Toothpaste C w/o described foam system C** |
|---|---|---|
| Time (sec.) | Ml | ml |
| 0 | 0 | 0 |
| 10 | 183 | 63 |
| 20 | 249 | 81 |
| 30 | 312 | 97 |
| 40 | 370 | 106 |
| 50 | 399 | 117 |
| 60 | 401 | 129 |
| 70 | 405 | 135 |
| 80 | 407 | 145 |
| 90 | 406 | 153 |
| 100 | 406 | 163 |

It can be seen in Table 6 that the strength of the foam forming system according to the invention performs the strongest in the presence of foam breaking components such as additional phosphates and zinc salts. This difference is maintained throughout the exposure time.

### Example 4: Comparison of single, dual and triple-surfactant toothpaste systems

Variations of three base toothpaste systems were prepared using one of three surfactant systems, as shown in the following Table 7:

**Table 7: Single, Dual and Triple Surfactant Systems**

| Formulation: | Toothpaste Base | Surfactants |
|---|---|---|
| Comparative D-1 | Multi-Protection | SLS |
| D-2 | Multi-Protection | SLS, CAPB |
| Comparative D-3 | Multi-Protection | SLS, CAPB, Poloxamer |
| Comparative E-1 | Enamel Protection | SLS |
| E-2 | Enamel Protection | SLS, CAPB |
| Comparative E-3 | Enamel Protection | SLS, CAPB, Poloxamer |
| Comparative F-1 | Whitening | SLS |
| F-2 | Whitening | SLS, CAPB |
| Comparative F-3 | Whitening | SLS, CAPB, Poloxamer |

Each Toothpaste Baste contains both potassium nitrate and sodium fluoride. Foam measurements were carried out as described in Example 1. The results are shown in Table 8 below.

The results demonstrate that both the dual- and triple-surfactant systems resulting in better foam generation than the single-surfactant system. However, it is unexpectedly found that foam generation is more rapid and stronger for the dual-surfactant system than for the triple-surfactant system during the first 50 seconds of test (which is the most critical to consumer acceptance). This trend is consistent across all three base compositions.

**Table 8: Foam Volume Data between Single, Dual and Triple Surfactant Systems**

| Time (Sec.) | Comp. D-1 | D-2 | Comp D-3 | Comp E-1 | E-2 | Comp E-3 | Comp F-1 | F-2 | Comp F-3 |
|---|---|---|---|---|---|---|---|---|---|
| 10 | 63 | 183 | 128 | 152 | 192 | 174 | 150 | 170 | 156 |
| 20 | 85 | 259 | 207 | 213 | 284 | 234 | 189 | 251 | 209 |
| 30 | 100 | 312 | 260 | 263 | 357 | 317 | 220 | 326 | 276 |
| 40 | 105 | 370 | 327 | 302 | 404 | 363 | 247 | 371 | 321 |
| 50 | 120 | 399 | 367 | 333 | 412 | 376 | 277 | 390 | 378 |
| 60 | 130 | 410 | 385 | 367 | 422 | 409 | 298 | 399 | 396 |

### Example 5: Foaming in Deionized Water Compared to Artificial Saliva

Toothpaste Compositions A and B, as well as their respective Reference Compositions A and B, as described in Examples 1 and 2 above, are further compared in their ability to generate foam when used with deionized water compared to with artificial saliva using the SITA foam tester. For each test, the foam volume was measured at various time points for each of Toothpaste A, Toothpaste B, Reference A and Reference B, in both deionized water and in artificial saliva. For each comparison (A or B) the difference in foam between the inventive toothpaste and the reference toothpaste was recorded. The results are shown in Table 9 below:

**Table 9: Difference in Foam Volume between Toothpaste A/B and Reference A/B**

| Time (Sec.) | Toothpaste B/ Reference B in Deionized Water | Toothpaste B/ Reference B in Artificial Saliva | Toothpaste A/ Reference A in Deionized Water | Toothpaste A/ Reference A in Artificial Saliva |
|---|---|---|---|---|
| 10 | -35 | 20 | 47 | 41 |
| 20 | 15 | 62 | 115 | 70 |
| 30 | 20 | 106 | 51 | 94 |
| 40 | -21 | 124 | -21 | 102 |
| 50 | -39 | 113 | -41 | 79 |
| 60 | -48 | 101 | -38 | 55 |

The results show that in artificial saliva, the use of the inventive surfactant system (either Toothpaste A or Toothpaste B) results in a consistent improvement in foaming over the reference surfactant system over the first 60 seconds of the experiment, with the greatest improvement in foaming occurring over the first 40 seconds. In contrast, when deionized water is used, it is found that the results are inconsistent: at some time points, the reference toothpaste has better foaming than the inventive toothpaste (shown by a negative value in the above table), while at other times point the reverse is seen. Thus, the surfactant systems according to the invention are uniquely tailored to produce clinically useful results in an artificial setting which mimics the oral cavity. Without being bound by theory, it is believed that natural components present in saliva may hinder the formation of adequate levels of foam by traditional surfactant systems, and these results show that the surfactant system according to the present invention overcomes this negative influence.

## Claims

1. An oral care composition comprising:
an anionic surfactant, wherein the anionic surfactant is sodium lauryl sulfate (SLS);
a cationic or amphoteric surfactant, wherein the cationic or amphoteric surfactant is cocamidopropyl betaine (CAPB); and
a potassium salt;
wherein the oral care composition does not comprise a non-ionic surfactant, wherein sodium lauryl sulfate is present in an amount of from 1.0 weight % to 3.0 weight %, and wherein cocamidopropyl betaine is present in an amount of from 0.30 weight% to 0.60 weight % of the composition.

2. The oral care composition of claim 1, further comprising a thickener.

3. The oral care composition of claim 2, wherein the thickener is selected from the group consisting of carboxymethyl cellulose, xanthan gum, and a methyl vinyl ether/maleic acid copolymer.

4. The oral care composition of claim 2, wherein the thickener is present in an amount of from 0.1 to 1.8 weight %; preferably from 0.2 to 1.00 weight %; most preferably from 0.4 to 0.65 weight %.

5. The oral care composition of claim 1, wherein the composition further comprises a zinc ion source.

6. The oral care composition of claim 5, wherein the zinc ion source is at least one selected from zinc citrate, zinc oxide, zinc acetate, zinc gluconate, zinc glycinate, zinc sulfate, zinc phosphate and sodium zinc citrate.

7. The oral care composition of claim 1, wherein the composition further comprises silica.

8. The oral care composition of claim 7, wherein the silica is at least one of an abrasive silica and/or a thickening silica, or combinations thereof.

9. The oral care composition of claim 1, wherein the composition further comprises a tartar control agent.

10. The oral care composition of claim 9, wherein the tartar control agent is tetrapotassium pyrophosphate and the total concentration of the tartar control agent is from 1 weight % to 4 weight %; preferably from 2 weight % to 3 weight %; preferably from 2.4 weight % to 2.6 weight %; 2.4 weight % or 2.5 weight %.

11. The oral care composition of claim 9, wherein the tartar control agent is tetrasodium pyrophosphate and the total concentration of the tartar control agent is from 0.2 to 0.8 weight %; preferably from 0.3 to 0.7 weight %; preferably from 0.4 to 0.6 weight %; or preferably 0.5 weight %.

12. The oral care composition of claim 1, wherein the potassium salt is potassium nitrate.

## Patentansprüche

1. Mundpflegezusammensetzung, umfassend:
ein anionisches oberflächenaktives Mittel, wobei das anionische oberflächenaktive Mittel Natriumlaurylsulfat (SLS) ist
ein kationisches oder amphoteres Tensid, wobei das kationische oder amphotere Tensid Cocamidopropylbetain (CAPB) ist; und
ein Kaliumsalz;
wobei die Mundpflegezusammensetzung kein nichtionisches Tensid umfasst, wobei Natriumlaurylsulfat in einer Menge von 1,0 Gewichts-% bis 3,0 Gewichts-% vorliegt, und wobei Cocamidopropylbetain in einer Menge von 0,30 Gewichts-% bis 0,60 Gewichts-% der Zusammensetzung vorliegt.

2. Mundpflegezusammensetzung nach Anspruch 1, weiterhin umfassend ein Verdickungsmittel.

3. Mundpflegezusammensetzung nach Anspruch 2, wobei das Verdickungsmittel ausgewählt ist aus der Gruppe bestehend aus Carboxymethylcellulose, Xanthangummi und einem Methylvinylether/Maleinsäure-Copolymer.

4. Mundpflegezusammensetzung nach Anspruch 2, wobei das Verdickungsmittel in einer Menge von 0,1 bis 1,8 Gewichts-%; vorzugsweise von 0,2 bis 1,00 Gewichts-%; am meisten bevorzugt von 0,4 bis 0,65 Gewichts-% vorliegt.

5. Mundpflegezusammensetzung nach Anspruch 1, wobei die Zusammensetzung weiterhin eine Zinkionenquelle umfasst.

6. Mundpflegezusammensetzung nach Anspruch 5, wobei die Zinkionenquelle mindestens eine ausgewählt aus Zinkcitrat, Zinkoxid, Zinkacetat, Zinkgluconat, Zinkglycinat, Zinksulfat, Zinkphosphat und Natriumzinkcitrat ist.

7. Mundpflegezusammensetzung nach Anspruch 1, wobei die Zusammensetzung ferner Silica umfasst.

8. Mundpflegezusammensetzung nach Anspruch 7, wobei das Silica mindestens eines von einem abrasiven Silica und/oder einem verdickenden Silica oder Kombinationen davon ist.

9. Mundpflegezusammensetzung nach Anspruch 1, wobei die Zusammensetzung weiterhin ein Mittel zur Kontrolle von Zahnstein umfasst.

10. Mundpflegezusammensetzung nach Anspruch 9, wobei das Mittel zur Kontrolle von Zahnstein Tetrakaliumpyrophosphat ist und die Gesamtkonzentration des Mittels zur Kontrolle von Zahnstein von 1 Gewichts-% bis 4 Gewichts-%; vorzugsweise von 2 Gewichts-% bis 3 Gewichts-%; vorzugsweise von 2,4 Gewichts-% bis 2,6 Gewichts-%; 2,4 Gewichts-% oder 2,5 Gewichts-% beträgt.

11. Mundpflegezusammensetzung nach Anspruch 9, wobei das Mittel zur Kontrolle von Zahnstein Tetranatriumpyrophosphat ist und die Gesamtkonzentration des Mittels zur Kontrolle von Zahnstein von 0,2 bis 0,8 Gewichts-%; vorzugsweise von 0,3 bis 0,7 Gewichts-%; vorzugsweise von 0,4 bis 0,6 Gewichts-%; oder vorzugsweise 0,5 Gesichts-% beträgt.

12. Mundpflegezusammensetzung nach Anspruch 1, wobei das Kaliumsalz Kaliumnitrat ist.

## Revendications

1. Composition de soins bucco-dentaires comprenant :
un tensioactif anionique, dans lequel le tensioactif anionique est le laurylsulfate de sodium (SLS) ;
un tensioactif cationique ou amphotère, dans lequel le tensioactif cationique ou amphotère est la cocamidopropyl bétaïne (CAPB) ; et
un sel de potassium ;
dans laquelle la composition de soins bucco-dentaires ne comprend pas de tensioactif non ionique, dans laquelle le laurylsulfate de sodium est présent en une quantité de 1,0 % en poids à 3,0 % en poids, et dans laquelle la cocamidopropyl bétaïne est présente en une quantité de 0,30 % en poids à 0,60 % en poids de la composition.

2. Composition de soins bucco-dentaires selon la revendication 1, comprenant en outre un épaississant.

3. Composition de soins bucco-dentaires selon la revendication 2, dans laquelle l'épaississant est choisi dans le groupe constitué par la carboxyméthylcellulose, la gomme xanthane et un copolymère éther méthylvinylique/acide maléique.

4. Composition de soins bucco-dentaires selon la revendication 2, dans laquelle l'épaississant est présent en une quantité de 0,1 à 1,8 % en poids ; de préférence de 0,2 à 1,00 % en poids ; de manière préférée entre toutes de 0,4 à 0,65 % en poids.

5. Composition de soins bucco-dentaires selon la revendication 1, dans laquelle la composition comprend en outre une source d'ions zinc.

6. Composition de soins bucco-dentaires selon la revendication 5, dans laquelle la source d'ions zinc est au moins une source choisie parmi le citrate de zinc, l'oxyde de zinc, l'acétate de zinc, le gluconate de zinc, le glycinate de zinc, le sulfate de zinc, le phosphate de zinc et le citrate de zinc sodique.

7. Composition de soins bucco-dentaires selon la revendication 1, dans laquelle la composition comprend en outre de la silice.

8. Composition de soins bucco-dentaires selon la revendication 7, dans laquelle la silice est au moins l'une d'une silice abrasive et/ou d'une silice épaississante, ou des combinaisons de celles-ci.

9. Composition de soins bucco-dentaires selon la revendication 1, dans laquelle la composition comprend en outre un agent de lutte contre le tartre.

10. Composition de soins bucco-dentaires selon la revendication 9, dans laquelle l'agent de lutte contre le tartre est le pyrophosphate tétrapotassique et la concentration totale de l'agent de lutte contre le tartre est de 1 % en poids à 4 % en poids ; de préférence de 2 % en poids à 3 % en poids ; de préférence de 2,4 % en poids à 2,6 % en poids ; de 2,4 % en poids ou de 2,5 % en poids.

11. Composition de soins bucco-dentaires selon la revendication 9, dans laquelle l'agent de lutte contre le tartre est le pyrophosphate tétrasodique et la concentration totale de l'agent de lutte contre le tartre est de 0,2 à 0,8 % en poids ; de préférence de 0,3 à 0,7 % en poids ; de préférence de 0,4 à 0,6 % en poids ; ou de préférence de 0,5 % en poids.

12. Composition de soins bucco-dentaires selon la revendication 1, dans laquelle le sel de potassium est le nitrate de potassium.
